(19) Europäisches Patentamt
European Patent Office
Office européen des brevets

(11) Publication number: 0 174 608
A1

# (12) EUROPEAN PATENT APPLICATION

(21) Application number: 85111225.0

(22) Date of filing: 05.09.85

(51) Int. Cl.⁴: C 12 N 15/00
C 12 P 19/34, C 12 N 5/00
C 12 P 21/02, C 07 K 13/00
C 07 H 21/04

(30) Priority: 13.09.84 US 650958

(43) Date of publication of application: 19.03.86 Bulletin 86/12

(84) Designated Contracting States: AT BE CH DE FR GB IT LI LU NL SE

(71) Applicant: THE BOARD OF TRUSTEES OF THE LELAND STANFORD JUNIOR UNIVERSITY
Encina 105 Stanford University
Stanford, California 94305(US)

(72) Inventor: Leavitt, John C.
1026 Henryton Road
Marriottsville Maryland 21104(US)

(72) Inventor: Kedes, Laurence H.
856 Allardice
Stanford California 94305(US)

(72) Inventor: Gunning, Peter W.
894 San Jude Avenue
Palo Alto California 94306(US)

(74) Representative: Glawe, Delfs, Moll & Partner
Patentanwälte
Postfach 26 01 62 Liebherrstrasse 20
D-8000 München 26(DE)

## (54) Beta-actin gene and regulatory elements, preparation and use.

(57) DNA sequences are provided for production of β-actin or untranslated regions of β-actin genes may be employed in conjunction with genes encoding for polypeptides for efficient expression in mammalian hosts. Particularly, the transcriptional and translational initiation and termination regions may be employed, by themselves or in combination with intron sequences for expression of various polypeptides in mammalian host cells.

./...



Croydon Printing Company Ltd.

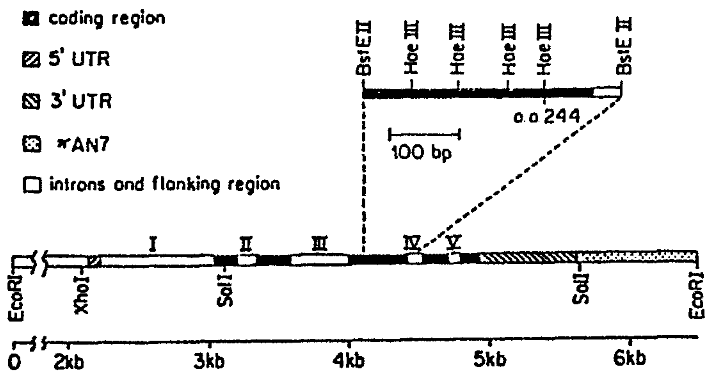
coding region
5' UTR
3' UTR
*AN7
introns and flanking region
BstEII
HaeIII
HaeIII
HaeIII
HaeIII
BstEII
a.a 244
100 bp
I
II
III
IV
V
EcoRI
XhoI
SalI
SalI
EcoRI
0
2kb
3kb
4kb
5kb
6kb

FIG._2.

# BETA-ACTIN GENE AND REGULATORY ELEMENTS, PREPARATION AND USE

## BACKGROUND OF THE INVENTION

### Field of the Invention

Expression in mammalian hosts offers many opportunities for the production of mammalian proteins, not available to unicellular microorganism hosts. By employing mammalian hosts, one can produce polypeptides which are properly processed, so as to be identical in composition to the native or wild-type protein, including glycosylation, methylation, methionine removal, N-terminal acetylation or formylation, and the like. Furthermore, there may be substantial efficiencies in translation, with concomitant reduction in mutation.

There is also a significant interest in naturally occurring proteins or alleles or mutants thereof, not only for use in research and therapy, but also for commercial purposes, where such polypeptides or proteins may serve in a variety of applications, such as polymeric units, additives, modifiers, bulking agents, or the like. In many situations it will be desirable that a mature polypeptide or protein is obtained, so that the final product has physical and chemical characteristics associated with the natural product.

It is therefore of interest to develop a portfolio of regulatory sequences which can be used in the transcription and translation of naturally occurring polypeptides and proteins including alleles, as well as mutants thereof or totally synthetic polypeptides and proteins based on modifications of naturally occurring analogs.

Furthermore, the protein β-actin serves a variety of structural purposes in the cell. The protein is particularly interesting for its ability to provide fibrous and film structures which can find commercial use as membranes, fibers, and the like.

Description of the Prior Art

Seed, Nuc. Acid Res. (1983) 11:2427-2446 describes a method for selecting genomic clones by homologous recombination. The nucleotide sequence for the mRNA derived from a β-actin cDNA clone is reported by Ponte et al., ibid (1984) 12:1687-1696. Vandekerckhove, Cell (1980) 22:893-899, reports coexpression of a mutant β-actin with two normal β-actins in a stably transformed human cell line. Ponte et al., Mol. Cell Biol. (1983) 3:1783-1791, report the presence of a large multi-pseudogene subfamily for β-actin. Ponte et al., ibid. also reports the 3'-untranslated regions of β-actin as isotype-specific. Nudel et al., Nucleic Acids Res. (1983) 11:1759-1771, predicted four intron sequences within the coding region of β-actin.

SUMMARY OF THE INVENTION

β-actin gene alleles including flanking DNA regulatory regions and introns are provided for expression of β-actin, as well as a source of regulatory DNA sequences including introns for use with other genes for expression in mammalian hosts. The 5'-untranslated region can be used as a transcriptional and translational region in combination with structural genes, where the structural gene may be modified by insertion of one or more introns for efficient processing of the initial transcription product to produce a mature messenger RNA. An homologous recombination technique is employed for isolation of complete β-actin genes capable of expression of β-actin in a mammalian host.

BRIEF DESCRIPTION OF THE FIGURES

Fig. 1 is a diagrammatic depiction of plasmid πAN7β1; and

Fig. 2 is a restriction endonuclease map and structure of the human β-actin gene M1(β1)-2.

DESCRIPTION OF THE SPECIFIC EMBODIMENTS

Polynucleotide sequences, combinations of the polynucleotide sequences, self-replicating constructs, host cells containing the constructs and methods employing the various polynucleotide compositions are provided for the expression of β-actin or other polypeptides in mammalian, particularly primate, cells. The sequences include the chromosomal gene for one or more allelic β-actins, including the flanking regions for the structural gene having transcriptional regulatory and translational initiation and termination sequences, coding sequences, intron sequences and cDNA encoding for one or more β-actin polypeptides. The sequences can be employed for expression of β-actin or fragments thereof, particularly fragments involving individual or combined exons, either combined as to β-actin exons or exons expressing other polypeptides. Also, the sequences may find use as probes for the determination of the presence of exons, introns, or flanking regions associated with β-actin in a mammalian, particularly primate, cell or other genes having homologous or partially homologous sequences.

A β-actin chromosomal DNA sequence including 5'- and 3'-flanking regions, introns and exons from a particular fetal source is set forth in the Experimental section. β-actins from other human sources will generally have at least 93 number percent of the same amino acids, usually at least 98 number percent, demonstrating substantial homology between the different β-actins. The β-actin structural gene including exons and introns will generally be about 3500 to 3600, more usually about 3550 nucleotides, inclusive of intron I, which is upstream from the initiation codon and intermediate the initiation codon and the TATA box. The complete cDNA sequence coding for β-actin will generally be of from about 2025 to 2125 nucleotides. The TATA box will generally be about 920 to 960, more usually about 940 nucleotides from the initiation codon. In the sequence in the Experimental section, the TATA box begins at -28 and terminates at -22, while the initiation codon begins at -916.

Intron I is subject to polymorphisms associated with different β-actin alleles. Intron I is indicated as beginning at nucleotide 79 and terminating at nucleotide 909. The polymorphic region is in the region of about 103 to 118 as numbered in the sequence. This region may be varied widely, where the sequence indicated has 16 base pairs (bp), other sequences may have up to 34bp or higher. The 5'-flanking region of β-actin may begin with the nucleotide designated as -28 in the sequence or be extended farther upstream, so that the TATA box, could be at a position 500, or even 3500 or more base pairs downstream from a restriction site in the chromosomal fragment, so as to provide for a greater non-transcribed region.

Alternatively, the TATA box may be only 25 to 50bp downstream from the initial nucleotide of the naturally occurring nucleotides present in the chromosomal sequence. Conveniently, all or a portion of intron I may be removed, desirably retaining the termini of intron I, where at least a portion of intron I is retained. Thus, one would wish to retain the splicing donor and acceptor sequences of intron I as well as at least one, preferably at least two, of the nucleotides flanking the intron, in order to favor accurate splicing. In this manner, transcriptional initiation and processing of the resulting messenger RNA may be efficiently achieved with DNA sequences coding for other than β-actin. Desirably, the DNA sequence from the terminus of intron I to the initiation codon can also be retained, so that any foreign DNA joined to that sequence would be joined to all or substantially all of the DNA upstream from the initiation codon of β-actin. Also, the 5'- sequence may extend into the coding region, usually not past the twelfth nucleotide, more usually not past the ninth nucleotide.

In some instances it may also be desirable to employ introns II, III, IV and/or V in a construction with a structural gene other than β-actin. In these situations, it would be desirable that the nucleotides immediately adjacent to the termini of the introns, which are part of the structural gene coding for the foreign protein have the same nucleotides, at least to the extent of one or two nucleotides, or be a transition, rather than a transversion, replacing a purine or pyrimidine with a purine or pyrimidine respectively. This may provide for enhanced accuracy in splicing. Any modification of the introns should preserve the AG and GT donor and acceptor splicing signals of the intron.

Any structure involving a foreign protein and one or more β-actin introns would involve fragmenting the structural gene encoding for the foreign protein, desirably of fragments of at least about 20 nucleotides, preferably of at least about 50 nucleotides, where the fragments can be conveniently ligated to the one or more introns. Conveniently, adapters may be used having appropriate termini, either cohesive or blunt, where the adapters may extend into the intron and/or exon.

The intron may be prepared by cloning the sequences, having derived them from β-actin genes, employing restriction enzyme digestion, exonuclease digestion, or the like, combinations of naturally occurring DNA sequences ligated to synthetic sequences, or combinations thereof. It may be desirable in some instances to mutagenize one or more nucleotides internal to an intron, so as to provide for a convenient restriction site, where relatively short adapters, generally from about 20 to 100 nucleotides may be prepared which can be used to join the intron to the exon to provide for splicing of two exons in proper reading frame. Alternatively, portions of the intron may be removed, for example, 10-90 percent of the base pairs, so long as the intron retains its capability of being excised in an appropriate host, e.g., mammalian, particularly mouse or primate.

Conveniently, the 3'- untranslated region of a β-actin gene may be employed for transcription and translational signals, particularly translational, since the structural gene will normally include one or more stop codons in reading frame with the mRNA coding sequence. Usually, the 3'- region will be at least 100bp, more usually at least 200bp, and may be 650bp or more depending upon the particular construction.

Expression of β-actin or foreign protein involving one or more introns may be achieved in a variety of ways in mammalian host cells. The coding construction involving the β-actin transcriptional initiation region, introns as appropriate and the structural gene present as a contiguous entity or as exons separated by one or more of the β-actin introns may be joined to an appropriate vector. By a vector is intended a replication system recognized by the intended host, where usually there is present one or more markers to ensure the stable maintenance of the DNA construct in the host.

Various replication systems include viral replication systems, such as retroviruses, simian virus, bovine papilloma virus, or the like. Alternatively, one may combine the DNA construct with a gene which allows for selection in a host. This gene can complement an auxotrophic host or provide protection from a biocide. Illustrative genes include thymidine kinase, dihydrofolate reductase, which provides protection from methotrexate, or the like. For the most part, markers will provide resistance to a biocide, e.g., G418, methotrexate, etc.; resistance to a heavy metal, e.g., copper; prototrophy to an auxotroph; or the like. Genes which find use include thymidine kinase, dihydrofolate reductase, metallothionein, and the like. In addition, the subject gene may be joined to an amplifiable gene, so that multiple copies of the structural gene of interest may be made. Depending upon the particular system, the gene may be maintained on an extrachromosomal element or be integrated into the host genome.

The foreign gene may come from a wide variety of sources, such as prokaryotes, eukaryotes, pathogens, fungi, plants, mammals, including primates, particularly humans, or the like. These proteins may include hormones, lymphokines, enzymes, capsid proteins, membrane proteins, structural proteins, growth factors and inhibitors, blood proteins, immunoglobulins, etc. The manner in which an individual DNA sequence coding for a protein of interest is obtained, divided into individual exons, and joined to the one or more introns and transcriptional and translational regulatory signals of β-actin will depend upon each individual polypeptide of interest, as well as the information available concerning the DNA sequence coding for such polypeptide.

The β-actin promoter or transcription system including the promoter may be used for the regulation of expression of other genes by regulating transcription of mRNA complementary to another mRNA or portion thereof. In effect, the β-actin promoter would regulate transcription of the nonsense strand or portion thereof of the gene whose expression is to be inhibited. Such inhibition may find use in making an auxotrophic host, inhibiting one pathway in favor of another metabolic pathway, reversing or enhancing oncogenic characteristics of a cell, or the like.

Introduction of the DNA into the host will vary depending upon the particular construction. Introduction can be achieved by transfection, transformation, transduction, or the like, as amply described in scientific literature. The host cells will normally be immortalized cells, that is, cells that can be continuously passaged in culture. For the most part, these cells will be neoplastic and may be any convenient mammalian cell, which is able to express the desired polypeptide, and where necessary or desirable, process the polypeptide, so as to provide a mature polypeptide. By processing is intended glycosylation, methylation, terminal acylation, e.g., formylation or acetylation, cleavage, or the like. In some instances it may be desirable to provide a leader sequence providing for secretion or directing the product to a particular locus in the cell. For secretion, the host should be able to recognize the leader sequence and the processing signal for peptidase cleavage and removal of the leader.

The isolation, cloning and verification of having a functional β-actin gene is complicated by the existence of numerous pseudogenes. Thus, strategies must be designed which ensure that the sequence obtained is a functional β-actin gene. Furthermore, by having a functional β-actin gene one can employ either untranslated or translated sequences as probes for determining the presence of other β-actin genes in a mammalian cell. The subject strategy for isolating and verifying the cloning of a β-actin gene included selecting genomic clones by homologous recombination.

The method employs a miniplasmid into which is inserted a fragment of either the untranslated region or translated region of a β-actin gene. Such a fragment may be obtained by isolation of a portion of the messenger RNA for β-actin. In the subject strategy, the fragment employed was from the 3'-untranslated region. The idea was that homologous recombination would occur with the greatest frequency with those sequences carrying the β-actin gene and having the highest degree of homology with the fragment present in the miniplasmid.

The recombination screen is conveniently carried out with a phage library as described by Seed, supra. A host is selected which is recombinant proficient and in which the viral vector of the library is unable to propagate. Therefore, only those viruses which undergo recombination with the miniplasmid will survive and can be isolated. Where the miniplasmid has a unique restriction site, and the same recognition sequence exists in the β-actin gene, it is feasible to screen fragments resulting from digestion of the recombinant phage to detect the presence of a fragment having the correct size. In this manner, pseudogenes may be distinguished from true genes.

Demonstration of β-actin alleles or mutants can be achieved by employing two different phage vectors, where each of the vectors have substantially different size packaging requirements, so that groups of fragments are separated by size. These hybrid phages are then combined with the miniplasmid containing the appropriate β-actin gene fragment for homologous recombination in an appropriate host. Those phage that propagate are then screened with an appropriate probe. It is found that the phage which includes fragments in the range of about 10 to 23kb provides a number of clones which include the complete β-actin gene, while the phage which includes fragments of 2 up to 13kb genomic DNA are found not to have clones with a complete β-actin gene, but rather appear to be pseudogenes.

The recombinant DNA produced β-actin can be used in a variety of ways. The protein is fibrous and can be used to make fibers or other structures. Furthermore, based on the differences between β- and γ-actins, one can modify the β-actin to change its structural properties. Thus, a variety of β-actins having different chemical and physical properties can be produced which can be used by themselves or in combination with other polyamides for the production of a wide variety of articles, such as fibers, films, formed objects, or the like. These pure fiber subunits will be synthesized in pro- and eukaryotes.

The DNA sequences which are provided can be used as probes, being used to detect mutational defects in β-actin and relating the mutational defects to cytoskeletal dysfunction as well as altered cellular phenotype.

The following examples are offered by way of illustration and not by way of limitation.

## EXPERIMENTAL

### Materials and Methods

General Methods.

Growth and transformation of *E. coli*, colony hybridization (Grunstein and Hogness, *Proc. Natl. Acad. Sci. USA* (1975) 72:3961-3965), and purification of plasmid DNA followed standard protocols as described previously (Childs *et al.*, *Dev. Biol.* (1979) 73:153-173). Preparation of Charon 4A and λgtWES phage recombinant DNA, agarose gels and hybridization blots, and the conditions used for hybridization were as described previously (Ponte *et al.*, *Mol. Cell Biol.* (1983) 3:1783-1791). Genomic DNA preparation from mammalian cells, DNA digestion with restriction enzymes, and hybridizations performed on nitrocellulose blots with dextran sulfate present were conducted as described by Ponte *et al.*, *Nature* (1981) 291:594-596. The human cell strains were grown and maintained as previously described (Leavitt and Kakunaga, *J. Biol. Chem.* (1980) 255:1650-1661).

Construction of the KD, HuT-14, and HuT-14T Human Gene Libraries.

Purified λ Charon 4A (Blattner *et al.*, *Science* (1977) 196:161-169) vector DNA (EcoRI arms), λgtWESλB (Leder *et al.*, *Science* (1977) 196:175-178) vector DNA (full length phage genome) and packaging extracts prepared from *E. coli* strains BHB2688 and BHB2690 were purchased from Amersham (Arlington Heights, IL). Fully or partially EcoRI digested fragments from genomic DNA, 2kb to 14kb or 10kb to 23kb, were purified from 5.5% agarose gels [Seakem HGT(P)] by adsorption to glass powder (Vogelstein and Gillespie, Proc. Natl. Acad. Sci. USA (1979) 76:615-619). Two to 14kb EcoRI DNA fragments were ligated to λgtWES DNA arms that were generated by EcoRI and SacI digestion of λgtWESλB DNA. Ten kb, or 12kb to 23kb, DNA EcoRI fragments (full or partial digests) were ligated into λ Charon 4A EcoRI arms. The ligation reaction consisted of 1 part human insert DNA and 3 parts vector DNA, 66mM Tris-HCl pH7.4, 5mM $MgCl_2$, 1mM ATP, 5mM dithiothreitol, 100μg bovine serum albumin (Fraction 5), and T4 ligase. Ligation reactions (13°C overnight) were always tested for completion by agarose gel analysis of reaction aliquots taken at the beginning and ends of the ligation reaction. Four μl of the ligation reaction products were mixed with the two packaging extracts and phage assembly was allowed for two hours at room temperature. Packaging reactions were then diluted with 0.5ml of phage dilution buffer (10mM Tris-HCl pH 7.4, 10mM $MgSO_4$, and 0.01% gelatin) followed immediately by 10μl of chloroform and storage at 4°C. Packaging titers were determined by infection of *E. coli* LE392.

Construction of the πAN7β1 Miniplasmid.

A 600bp EcoRI to BamHI fragment of the cDNA (β-actin 3'UTR sequence) insert in pHFβA-3'UT (Ponte *et al*. (1983), *supra*) was purified by gel electrophoresis and adsorption to glass powder and then ligated to the EcoRI to BamHI large fragment (alkaline phosphatase treated) of plasmid πAN7. (A derivative of πVX (Seed (1983), *supra*, which contains the tyrosinyl suppressor tRNA gene (SupF) and a polylinker with eight restriction sites. Also, the colicin E1 replicon is present, see Fig. 1. The 600bp 3'-untranslated sequences (3'UTR) EcoRI-BamHI fragment is inserted into the restriction sites of the polylinker.) *E. coli* W3110(p3) (need citation) was transformed with the ligation mixture and plasmid DNA from individual ampicillin-resistant ($Amp^r$) and tetracycline-resistant ($Tet^r$) colonies was amplified. The structure of πAN7β1 (the 3'-UTR sequence is oriented so that the SalI site in the miniplasmid is placed near the junction between the 3'-terminus of the 3'-UTR and the miniplasmid) was confirmed by restriction analysis and DNA blotting experiments.

Selection of πAN7β1 Recombinant λ Phage.

A recombination screen (Seed (1983), supra; DiMaio et al., Mol. Cell Biol. (1984) 4:340-350) to isolate phage containing DNA homologous to the 3'UTR sequence in πAN7β1 from a highly amplified gene library (Maniatis et al., Cell (1978) 15:687-701) was performed. The library was prepared by ligation of partial EcoRI digests of DNA derived from a human fetus to the Charon 4A vector. Phage stocks were prepared by infecting bacteria carrying πAN7β1 with $10^6$ PFU of the Charon 4A library. Phage able to form plaques on W3110($Su^-$) bacteria were present in the lysate at frequencies between $10^{-7}$ and $10^{-9}$. See Table 1.

The presence of actin coding sequences as well as the 3'UTR and plasmid vector sequence in these rare clones was confirmed by blotting experiments on Southern transfers of restriction endonuclease-digested DNA isolated after propagation of phage from individual plaques.

Recombination screens were then performed as above on unamplified phage in packaging reactions that were generated by ligation of EcoRI digested HuT-14 and HuT-14T DNA ligated to the λgtWES vector arms (Leder et al. (1977), supra) and phage packaging reactions that were generated by ligation of EcoRI digested KD, HuT-14 and HuT-14T DNA (cell line sources) ligated to the Charon 4A vector arms. Frequencies of recovery of library phage clones by recombination selection that contain the β-actin gene are presented in Table 2.

TABLE 1: Selection of Phage Clones Containing Actin Sequences by πAN7β1 Recombination

| Recombination Trial | Clone | Frequency of Recovery | Genomic EcoRI Fragment with Coding[a,b] Size(kb) | 5'coding sequence | 3'coding sequence | 3'UTR sequence | Addition EcoRI Fragments with only 3'UTR seq. |
|---|---|---|---|---|---|---|---|
| I | M1(β1)-1 | $1.3x10^{-7}$ | 5.0kb | - | + | + | 1.4kb, 1.5kb |
| | M1(β1)-2 | $3.3x10^{-9}$ | 6.6kb | + | + | + | 7.1kb, 1.5kb |
| II | M4(β9)-1 | $3.8x10^{-8}$ | 2.2kb | - | + | + | 1.0kb |
| | M4(β9)-2 | $7.4x10^{-8}$ | 5.8kb | - | + | + | 1.1kb |
| | M4(β9)-3 | $1.8x10^{-8}$ | 2.0kb | - | + | + | 0.7kb |

[a] the 5' coding probe was an AvaI restriction fragment for amino acids 1 through 98 of human skeletal actin (Gunning, et al., Mol. Cell. Bio. (1983) 3:787-795);

[b] the 3' coding probe was a KpnI restriction fragment for amino acids 301 through 374 and part of the 3'UTR sequence of a chick β-actin cDNA (Engel, et al., Proc. Natl. Acad. Sci. USA (1981) 78:4674-4678).

TABLE 2: Components of the Human HuT-14/HuT-14T Gene Libraries

| Source of Human DNA | Vector | Sizes of EcoRI Fragments Cloned | Human Haploid Genome Equivalents | Frequency of Clones with β-Actin Sequence per $10^5$ Phage Recombinants |
|---|---|---|---|---|
| HuT-14 | λgtWES | 2kb to 14kb | 3.8 | 8.3 [b] |
| HuT-14T | λgtWES | 2kb to 14kb | 9.0 | 7.1 [b] |
| HuT-14 | Charon 4A | 12kb to 23kb | 30.0 [a] | 4.0 [b] |
| HuT-14T | Charon 4A | 10kb to 23kb | 30.0 [a] | 5.0 [b] |
| HuT-14T | Charon 4A | 10kb to 23kb (partial digest fragments) | 0.6 | 5.9 [c] |

[a] calculated from the frequency of recovery of the β-actin gene

[b] the frequency of recovery of library phage clones containing β-actin sequences selected by πAN7β1 recombination

[c] the frequency of recovery of library phage clones containing β-actin sequences selected by <u>in situ</u> plaque hybridization with the 3'UTR probe

A recombination was performed in which $10^6$ PFU of library phage were amplified by infection in the recombinant proficient E. coli strain WoP3πAN7β1. Lytic progeny phage from the amplification were used to infect a host strain (WoP3Sup0) in which Charon 4A phage do not propagate, so that no lytic plaques are produced in the absence of recombination. Infection of the host produced plaques at a consistent frequency between $10^7$-$10^9$ of its true titer. All phage that were isolated contained actin coding sequences and had undergone recombination with the πAN7β1 plasmid.

Five distinct phage clones were selected as set forth in Table 1, with the sizes of the EcoRI fragments containing coding or non-coding 3'-UTR sequences indicated. In the recombination trial, 50 of the 51 plaques isolated were identical and designated M1(β1)-1. In addition to three EcoRI fragments that contained actin coding sequences (5.0kb, 1.4kb, 1.5kb), one additional EcoRI fragment (3.5kb) which lacked an actin coding sequence was common to all 50 isolates. A single additional plaque (M1(β1)-2) contained a different phage with a different set of EcoRI fragments: three fragments contained actin sequences (6.6kb, 7.1kb and 1.5kb) and two fragments lacked actin sequences (2.0kb and 1.2kb).

A second recombination trial produced three additional and still different recombinant clones (Table 1). The recovery of different plaque types during independent trials was interpreted as being a result of the skewed nature of the human lambda library as well as the degree of sequence similarity between the πAN7β-actin insert and the various genomic β-actin sequences.

M1(β1)-2 was distinguished from the other isolates in that it hybridized to a probe that contained the 5'-actin coding sequence (codons 1-98). SalI digestion of M1(β1)-2 generates a 2500bp fragment that contains most of the coding sequences for β-actin plus the 3'UTR sequence. The nucleotide sequence of the fragment was determined, which confirmed the position of the *SalI* site at codon 10 and the existence of four intron regions, the sum of whose lengths is 731bp. Furthermore, the nucleotide sequences of the coding regions of M1(β1)-2 was shown to be identical to the β-actin cDNA sequence. Restriction mapping of lambda clone M1(β1)-2 demonstrated the presence of the β-actin sequence on a 12.2kb genomic fragment which divided into two *EcoRI* fragments of 6.6 and 7.1kb by πAN7β1 recombination.

Size fractionated *EcoRI* fragments ranging from 10 to 12kb and larger from HuT-14 and HuT-14T DNA were used to prepare recombinant phage. See Table 2. Amplification aliquots ($10^4$ packaging events) were first screened by πAN7β1 recombinant selection to determine which library aliquots contained any β-actin genes or pseudogenes. Those library aliquots that contained β-actin 3'UTR sequences were rescreened by conventional *in situ* plaque hybridization to select clones that hybridized to the 3'UTR probe. Following purification, each β-actin clone was recombined with πAN7β1 and the recombinant forms examined by *EcoRI* and *SalI* restriction endonuclease digestion and the resulting DNA fragments hybridized with intron I, 3'UTR and coding probes to fully assess their identity and relatedness. Table 3 summarizes the characteristics of each clone that was isolated in this way.

TABLE 3: Clones of β-Actin Sequences Isolated from Libraries of 10kb to 23kb EcoRI Fragments

| Library Source | Clone | Genomic EcoRI Fragment size (kb) | Distance from the 5'EcoRI site to 3'end of the 3'UTR (SalI site) (kb) [a] | 2.5kb SalI Fragment [b] | Hybridization to the Intron I Probe |
|---|---|---|---|---|---|
| HuT-14T fully digested 10 to 23kb EcoRI fragments in Charon 4A | 14Tβ-15 | 13.8 | 6.6 | + | + |
| | 14Tβ-16 | 13.8 | 6.6 | + | + |
| | 14Tβ-17 | 13.8 | 6.6 | + | + |
| | 14Tβ-18 | 11.0 | 4.3 | - | + |
| | 14Tβ-19 | 13.8 | 6.6 | + | + |
| | 14Tβ-20 | 13.8 | 6.6 | + | + |
| | 14Tβ-21 | 13.8 | 6.6 | + | + |
| | 14Tβ-22 | 14.2 | 8.5 | - | + |
| | 14Tβ-23 | 13.8 | 6.6 | + | + |
| | 14Tβ-24 | 13.8 | 6.6 | + | + |
| HuT-14 fully digested 10 to 23kb EcoRI fragments in Charon 4A | 14β-25 | 18.5 | 14.6 | - | - |
| | 14β-26 | 14.4 | 6.0 | + | + |
| | 14β-27 | 13.8 | 6.6 | + | + |
| | 14β-28 | nd | 14.1 | - | - |
| | 14β-29 | 13.8 | 6.6 | + | + |
| | 14β-30 | 13.8 | 6.6 | + | + |
| | 14β-31 | 13.8 | 6.6 | + | + |
| | 14β-32 | 13.8 | 6.6 | + | + |

CONTINUED ON NEXT PAGE

TABLE 3: Clones of β-Actin Sequences Isolated from Libraries of 10kb to 23kb EcoRI Fragments (CONTINUED FROM PREVIOUS PAGE)

| Library Source | Clone | Genomic EcoRI Fragment size (kb) | Distance from the 5'EcoRI site to 3'end of the 3'UTR (SalI site) (kb) [a] | 2.5kb SalI Fragment [b] | Hybridization to the Intron I Probe |
|---|---|---|---|---|---|
| KD fully digested 10 to 23kb EcoRI fragments in Charon 4A | KDβ-1 | 13.8 | 6.6 | + | + |
| HuT-14T partially digested 10 to 23kb EcoRI fragments in Charon 4A | 14Tβ-1 | 5.3 | 4.3 | - | - |
| | 14Tβ-2 | 10.5 | 7.9 | - | - |
| | 14Tβ-4 | 4.3 | 3.8 | - | - |
| | 14Tβ-5 | 8.1 | nd | - | - |
| | 14Tβ-12 | 2.9 | nd | - | - |

[a] πAN7β1 recombinant phage clones were constructed with plaque pure clones selected by in situ plaque hybridization with the 3'UTR probe; prior to recombination each clone contained a single human EcoRI fragment; following recombination two EcoRI fragments were generated by insertion of πAN7β1 into the genomic EcoRI fragment; the sizes of the two EcoRI fragments generated and identification of the fragment containing coding and intron I sequences revealed the position of the β-actin sequence within the genomic EcoRI fragment

[b] the 2.5kb SalI fragment is generated as a result of πAN7β1 insertion during recombination and is characteristic of the β-actin gene (Fig. 1)

In total, eight of ten isolates from HuT-14T DNA and five of eight isolates from HuT-14 DNA contained a β-actin gene similar to the that found in M1(β1)-2, each of these separate clones hybridizing strongly to the intron probe. In addition, the πAN7β1 recombinants contained the characteristic 2.5kb SalI restriction endonuclease fragment carrying the β-actin coding, intron and 3'UTR sequences. The size of the uninterrupted genomic fragment for these clones was about 13.8kb.

The EcoRI restriction endonuclease fragment carrying the β-actin gene, including its introns, in the πAN7β1 KD, HuT-14 and HuT-14T recombinants is 8.2kb long (Table 3). By contrast, EcoRI fragments bearing the β-actin gene in M1(β1)-2, derived from the human fetal DNA library, appear to be only 6.6kb long.

To determine whether the differences in fragment lengths was due to a restriction site polymorphism or represented parologous alleles, EcoRI digestion fragments of the three of the πAN7β1 recombinant β-actin clones from HuT-14 DNA (14β-27(β1), 14β-29(β1), and 14β-30(β1)) and the fetal gene clone M1(β1)-2 were subcloned into pBR322. These subclones were digested with EcoRI and the resulting fragments separated by agarose gel electrophoresis. The blots were first hybridized to the β-actin intron I probe and then the same blot hybridized with the β-actin 3'UTR probe. The intron probe hybridized to the 8.2kb EcoRI fragment of 14β-27(β1), 14β-29(β1), and 14β-30(β1) and the 6.6kb EcoRI fragment of M1(β1)-2. By contrast, the 3'UTR probe hybridized at the 7.1kb EcoRI DNA fragment, common to all four clones, as well as to the 8.2kb or 6.6kb EcoRI fragments containing the intron I sequences. This result indicates that the genes isolated from HuT-14 and HuT-14T DNA differ from the fetus-derived gene in M1(β1)-2 in the location of an EcoRI site in the genomic DNA flanking the 5' region of the β-actin gene. All 13 independent πAN7β1 recombinant clones derived from both

HuT libraries and one additional clone derived from the KD cell DNA library have an identical arrangement with regard to the positions of flanking EcoRI sites. The uninterrupted EcoRI fragment and the corresponding non-πAN7 recombinant clones is 13.8kb, from which it is concluded that the β-actin gene probably resides on a 13.8kb genomic EcoRI fragment.

The sequences derived from the gene in M1(β1)-2 and from a cDNA clone (Ponte et al., Nuc. Acids Res. (1984) 12:1687-1696) show that codons 243, 244, and 245 (-Asp-Gly-Gln-) were encoded by GAC GGC CAA. Since the first β-actin mutation of HuT-14 resulted in an exchange of the glycine (codon 244) for an aspartic acid residue, the predicted sequence for codon 244 after the mutation is GAC. The unmutated sequence GGCC (codons 244 and 245) is a restriction site for the endonuclease HaeIII, a site which should be absent in mutant copies of the gene from HuT-14 and HuT-14T. BstEII sites flank the mutation site and cleave between the codon 158 and 159 and at a site 38bp into intron IV respectively. This BstEII fragment (366bp) was isolated from the DNA of three plasmid subclones of the HuT-14 πAN7β1 derived β-actin genes (the 8.2kb EcoRI fragment from 14β-27(β1), 14β-29(β1), and 14β-30(β1) and three additional plasmid subclones from non-πAN7 derived HuT-14T β-actin genes (the 13.8kb EcoRI fragment from 14Tβ-17, 14Tβ-21 and 14Tβ-24). Within this BstEII fragment there are HaeIII sites at codons 182, 203, 204, 228 and 244, the site of the mutation (Fig. 2). Digestion of the BstEII fragment from the wild-type β-actin gene with HaeIII generates five restriction fragments of 71, 65, 72, 52 and 106bp, respectively, whereas the mutated gene missing the HaeIII site at codon 244 should produce four restriction fragments of 71, 65, 72 and 158bp. Four of six clones from HuT-14 (clones 14β-27(β1) and 14β-29(β1)) and HuT-14T (clones 14Tβ-21 and 14Tβ-24) exhibited the 158bp HaeIII-BstEII fragment indicative of copies of the gene mutated at codon 244. The two remaining clones 14β-30(β1) and 14Tβ-17 exhibited the wild-type digestion pattern indicative of the normal unmutated gene. Thus, the β-actin genes cloned from the HuT-14 and HuT-14T DNA libraries represent both the wild-type and mutant alleles. Furthermore, the presence of the predicted mutation in one of the alleles formally proves that these genes, and not the other EcoRI β-actin coding fragments, are the expressed β-actin genes in these human fibroblast strains. The sequences of the genes carrying the mutations confirms that these genes are expressed.

A β-actin expression vector providing the β-actin promoter region, a polylinker and a polyadenylation signal was constructed where the expression construct was present on a vector having a bacterial origin of replication, as well as a marker for selection in a mammalian host.

A 4.3kb EcoRI-AluI fragment containing 3.4kb of the DNA upstream of the CAP site plus 5'-untranslated region plus IVSI terminating at the splice junction was isolated such that the sequence terminates 6bp from the initiation codon; this fragment was obtained from clone 14Tβ17. Plasmid pSP64 (Melton, et al., Nucl. Acids Res. (1984) 12:7035-7056) was digested with BamHI, the overhang filled in with the Klenow fragment, followed by digestion with EcoRI and ligation to the EcoRI-AluI β-actin fragment. The resulting plasmid was first digested with HindIII, the HindIII site filled in with the Klenow fragment, followed by digestion with EcoRI to provide an EcoRI-flush HindIII fragment containing the β-actin sequence.

Plasmid pcDV1 (Okayama and Berg, Mol. Cell. Biol. (1983) 3:280-289) was employed for the SV40 polyadenylation signal corresponding to a BamHI-BclI (map positions 0.145 to 0.19) fragment. The SalI and AccI sites were destroyed by sequentially digesting the plasmid with the appropriate restriction enzyme, removing

the overhang with S1 nuclease and ligating the resulting flush ends. The resulting plasmid was then digested with *XhoI*, which is present proximal to the 5'-terminus of the SV40 polyadenylation signal containing fragment, the *XhoI* site filled in, followed by digestion of linear fragments with *EcoRI* to provide an *EcoRI*-flush *XhoI* fragment. This fragment was then ligated with the *EcoRI*-flush *HindIII* fragment containing the β-actin sequences. The resulting plasmid was digested with *EcoRI* and *ClaI* to provide a linear fragment containing the promoter region from β-actin, a polylinker sequence, and the SV40 polyadenylation site.

Plasmids pSV2-neo (Southern and Berg, *J. Mol. Appl. Genet.* (1982) 1:327-341) and pSV2-gpt (Mulligan and Berg, *Proc. Natl. Acad. Sci. USA* (1981) 78:2072-2076) were each sequentially digested with *HindIII* and *BamHI*, followed by filling in the overhang with the Klenow fragment and recircularizing. The resulting modified plasmids were then digested with *PvuII* and *EcoRI* to provide new fragments having the SV40 origin and SV40 promoter, and either the neomyosin phosphoryl transferase gene or xanthine guanine phosphoribosyl transferase gene, followed by the SV40 polyadenylation site.

The neo fragment and gpt fragments were inserted into the *ClaI*-*EcoRI* fragment to provide expression vectors which could be selected by G418 resistance or resistance to aminopterin and mycophenolic acid, respectively. The vectors were then ready for use for insertion of a gene for expression in a mammalian host under the regulatory control of the β-actin promoter and for selection of recipient mammalian cells.

The following represents the complete sequence for the β-actin gene, including flanking regions, which include the promoter region and the termination region, as well as the introns, indicating the splicing sites for the introns.

```
CCCAGCACCC CAAGGCGGCC AACGCCAAAA CTCTCCCTCC TCCTCTTCCT CAATNCTCGC TCTCGCTCTT TTTTTTTTTC GCAAAAGGAG GGGAGAGGGG GTAAAAAAAT GCTGCACTGT  -121
CGGCGAAGCC GGTGAGTGAG CGGCGCGGGG CCAATCGCGT GCGCCGTTCC GAAAGTTGCC TTTTATGGCT CGAGCGGCCG CGGCGGCGCC CTATAAAACC CAGCGGCGCG ACGCGCCACC  -1
1 ACCGCCGA GACCGCGTCC GCCCCGCGAG CACAGAGCCT CGCCTTTGCC GATCCGCCGC CCGTCCACAC CCGCCGCCAG  GTAAGCCCG GCCAGCCGAC CGGGGCATGC GGCCGCGGCC  117
CCTTCGCCCG TGCAGAGCCG CCGTCTGGGC CGCAGCGGGG GGCGCATGGG GGGGGAACCG GACCGCCGTG GGGGGCGCGG GAGAAGCCCC TGGGCCTCCG GAGATGGGGG ACACCCCACG  237
CCAGTTCGGA GGCGCGAGGC CGCGCTCGGG AGGCGCGCTC CGGGGGTGCC GCTCTCGGGG CGGGGGCAAC CGGCGGGGTC TTTGTCTGAG CCGGGCTCTT GCCAATGGGG ATCGCAGGGT  357
GGGCGCGGCG TAGCCCCCGC CAGGCCCGGT GGGGGCTGGG GCGCCATGCG CGTGCGCGCT GGTCCTTTGG GCGCTAACTG CGTGCGCGCQ GGGAATTGGC GCTAATTGCG GCTGCGGCCQ  477
GGGACTCAAG GCGCTAATTG CGGCTGCGTT CTGGGGCCCG GGGTGCCGCG GCCQGGGCQG GGGCGAAGGC GGGCTCGGTC GGAAGGGGTG GGGTCGCCGC GGCTCCCGGG CGCTTGCGCA  597
CTTCCTGCCC GAGCCGCQGG CCGCCCGAGG GTGTGGCCGC TGCGTGCGCG CGCGCGACCC GGCGCTGTTT GAAQCGGGCG GAGGCGGGGC TGGCGCCCGG TTGGGAGGGG GTTGGGGCCT  717
GGCTTCCTGC CGCGCGCCGC GGGGACGCCT CCGACCAGTG TTTGCCTTTT ATGGTAATAA CGCGCCGGCC CGGCTTCCTT TATCCCCAAT CGTGCGCGCG CCGGCGCCCC CTAGCGGCCT  837
AAGGACTCGG CGCGCCGGAA GTGGCCAGGG CGGGGGCGAC TTCGGCTCAC AGCGCGCCCG GCTATTCTCG CAG CTCACC ATG GAT GAT GAT ATC GCC GCG CTC GTC GTC GAC  949
                                                                                           MET Asp Asp Asp Ile Ala Ala Leu Val Val Asp
                                                                                                                                    10

AAC GGC TCC GGC ATG TGC AAG GCC GGC TTC GCG GGC GAC GAT GCC CCC CGG GCC GTC TTC CCC TCC ATC GTG GGG CGC CCC AGG CAC CAG GTAGGGGAGCT  1050
Asn Gly Ser Gly Met Cys Lys Ala Gly Phe Ala Gly Asp Asp Ala Pro Arg Ala Val Phe Pro Ser Ile Val Gly Arg Pro Arg His Gln
                                 20                                     30                                     40

GGCTGGGTGG GGCAGCCCCG GGAGCGGGCG GGAGGCAAGG GCGCTTTCTC TGCACAGGAG CCTCCCGGTT TCCGGGGTGG GGGCTGCGCC GCTGCTCAGG GCTTCTTGTC CTTTCCTTCC  1170
CAG GGC GTG ATG GTG GGC ATG GGT CAG AAG GAT TCC TAT GTG GGC GAC GAG GCC CAG AGC AAG AGA GGC ATC CTC ACC CTG AAG TAC CCC ATC GAG CAC  1269
    Gly Val Met Val Gly Met Gly Gln Lys Asp Ser Tyr Val Gly Asp Glu Ala Gln Ser Lys Arg Gly Ile Leu Thr Leu Lys Tyr Pro Ile Glu His
                                     50                                     60                                     70

GGC ATC GTC ACC AAC TGG GAC GAC ATG GAG AAA ATC TGG CAC CAC ACC TTC TAC AAT GAG CTG CGT GTG GCT CCC GAG GAG CAC CCC GTG CTG CTG ACC  1368
Gly Ile Val Thr Asn Trp Asp Asp Met Glu Lys Ile Trp His His Thr Phe Tyr Asn Glu Leu Arg Val Ala Pro Glu Glu His Pro Val Leu Leu Thr
                         80                                     90                                     100

GAG GCC CCC CTG AAC CCC AAG GCC AAC CGC GAG AAG ATG ACC CAG  GTGAGTGGCC  CGCTACCTCT  TCTGGTGGCC  GCCTCCCTCC  TTCCTGGCCT  CCCGGAGCTG  1473
Glu Ala Pro Leu Asn Pro Lys Ala Asn Arg Glu Lys Met Thr Gln
            110                                     120

CGCCCTTTCT CACTGGTTCT CTCTTCTGCC GTTTTCCGTA GGACTCTCTT CTCTGACCTG AGTCTCCTTT GGAACTCTGC AGGTTCTATT TGCTTTTTCC CAGATGAGCT CTTTTTCTGG  1593
TGTTTGTCTC TCTGACTAGG TGTCTAAGAC AGTGTTGTGG GTGTAGGTAC TAACACTGGC TCGTGTGACA AGGCCATGAG GCTGGTGTAA AGCGGCCTTG GAGTGTGTAT TAAGTAGGCG  1713
CACAGTAGGT CTGAACAGAC TCCCCATCCC AAGACCCCAG CACACTTAGC CGTGTTCTTT GCACTTTCTG CATGTCCCCC GTCTGGCCTG GCTGTCCCCA GTGGCTTCCC CAGTGTGACA  1833
TGGTGCATCT CTGCCTTACAG  ATC ATG TTT GAG ACC TTC AAC ACC CCA GCC ATG TAC GTT GCT ATC CAG GCT GTG CTA TCC CTG TAC GCC TCT GGC CGT ACC  1935
                        Ile Met Phe Glu Thr Phe Asn Thr Pro Ala Met Tyr Val Ala Ile Gln Ala Val Leu Ser Leu Tyr Ala Ser Gly Arg Thr
                                                         130                                     140

ACT GGC ATC GTG ATG GAC TCC GGT GAC GGG GTC ACC CAC ACT GTG CCC ATC TAC GAG GGG TAT GCC CTC CCC CAT GCC ATC CTG CGT CTG GAC CTG GCT  2034
Thr Gly Ile Val Met Asp Ser Gly Asp Gly Val Thr His Thr Val Pro Ile Tyr Glu Gly Tyr Ala Leu Pro His Ala Ile Leu Arg Leu Asp Leu Ala
    150                                     160                                     170                                     180

GGC CCG GAC CTG ACT GAC TAC CTC ATG AAG ATC CTC ACC GAG CGC GGC TAC AGC TTC ACC ACC ACG GCC GAG CGG GAA ATC GTG CGT GAC ATT AAG GAG  2133
Gly Arg Asp Leu Thr Asp Tyr Leu Met Lys Ile Leu Thr Glu Arg Gly Tyr Ser Phe Thr Thr Thr Ala Glu Arg Glu Ile Val Arg Asp Ile Lys Glu
                                 190                                     200                                     210
```

```
AAG CTG TGC TAC GTC GCC CTG GAC TTC GAG CAA GAG ATG GCC ACG GCT GCT TCC AGC TCC TCC CTG GAG AAG AGC TAC GAG CTG CCT GAC GGC CAG GTC  2232
Lys Leu Cys Tyr Val Ala Leu Asp Phe Glu Gln Glu Met Ala Thr Ala Ala Ser Ser Ser Ser Leu Glu Lys Ser Tyr Glu Leu Pro Asp Gly Gln Val
                    220                                     230                                     240

ATC ACC ATT GGC AAT GAG CGG TTC CGC TGC CCT GAG GCA CTC TTC CAG CCT TCC TTC CTG G  GTGAGTGGAG ACTGTCTCCC GGCTCTGCCT GACATGAGGG TTAC  2337
Ile Thr Ile Gly Asn Glu Arg Phe Arg Cys Pro Glu Ala Leu Phe Gln Pro Ser Phe Leu
            250                                 260

CCCTCGGGGC TGTGCTGTGG AAGCTAAGTC CTGCCCTCAT TTCCCTCTCAG  GC ATG GAG TCC TGT GGC ATC CAC GAA ACT ACC TTC AAC TCC ATC ATG AAG TGT GAC  2444
                                                      Gly Met Glu Ser Cys Gly Ile His Glu Thr Thr Phe Asn Ser Ile Met Lys Cys Asp
                                                                  270                                     280

GTG GAC ATC CGC AAA GAC CTG TAC GCC AAC ACA GTG CTG TCT GGC GGC ACC ACC ATG TAC CCT GGC ATT GCC GAC AGG ATG CAG AAG GAG ATC ACT GCC  2543
Val Asp Ile Arg Lys Asp Leu Tyr Ala Asn Thr Val Leu Ser Gly Gly Thr Thr Met Tyr Pro Gly Ile Ala Asp Arg Met Gln Lys Glu Ile Thr Ala
                290                                     300                                     310

CTG GCA CCC AGC ACA ATG AAG ATC AAG GTGGGTGTCT TTCCTGCCTG AGCTGACCTG GGCAGGTCAG CTGTGGGGTC CTGTGGTGTG TGGGGAGCTG TCACATCCAG GGTCCTC  2657
Leu Ala Pro Ser Thr Met Lys Ile Lys
    320

ACTGCCTGTC CCCTTCCCTC CTCAG ATC ATT GCT CCT CCT GAG CGC AAG TAC TCC GTG TGG ATC GGC GGC TCC ATC CTG GCC TCG CTG TCC ACC TTC CAG CAG  2760
                            Ile Ile Ala Pro Pro Glu Arg Lys Tyr Ser Val Trp Ile Gly Gly Ser Ile Leu Ala Ser Leu Ser Thr Phe Gln Gln
                                    330                                     340                                     350
                                                                                                                                      2826
ATG TGG ATC AGC AAG CAG GAG TAT GAC GAG TCC GGC CCC TCC ATC GTC CAC CGC AAA TGC TTC TAG
Met Trp Ile Ser Lys Gln Glu Tyr Asp Glu Ser Gly Pro Ser Ile Val His Arg Lys Cys Phe Ter
                        360                                     370
```

The sequence that codes for mRNA begins at nucleotide position 1, the nucleotides being numbered relative to the A of the cap site. The first intron begins at about nucleotide 79 and ends atposition 910, and is followed by a six member nucleotide sequence that codes for further 5' untranslated mRNA before translation commences at nucleotide 917. Nucleotides 103 to 118 in intron I include a polymorphic region. In the human fibroblast gene derived from clones 14β27 and 14Tβ24, this polymorphic region is replaced by the sequence CAGGCGGCTCACGPCCCGPCCGGCAGGCPCCGGAC. For the human fibroblast gene derived from clone 14Tβ21, the polymorphic sequence is replaced by CAGCGGCCAGCGCCGCAGGCCGCGGCCC. Also, a 30 base-pair highly conserved, intervening sequence exists at bases 752 to 781. Where the exact identity of a base has not been verified, P indicates a purine, Q refers to a pyrimidine, and N refers to any nucleotide. The amino acid sequence is numbered according to Lu and Elzinga, <u>Biochemistry</u> (1977) 5801-5806.

It is evident from the above results, that DNA sequences are provided which can be used for detecting polymorphisms, alleles and mutants of β-actin. In addition, the fragments of the sequences can be obtained by appropriately restricting the DNA, isolating individual fragments, and using the fragments as regulatory signals or introns. As indicated, DNA sequences from various structural genes may be joined to one or more introns, as well as the transcriptional regulatory sequence for β-actin to provide for constitutive efficient production of polypeptides of interest in appropriate mammalian hosts.

Although the foregoing invention has been described in some detail by way of illustration and example for purposes of clarity of understanding, it will be obvious that certain changes and modifications may be practiced within the scope of the appended claims.

WHAT IS CLAIMED IS:

1. A genomic DNA sequence of less than 15kb encoding for a human β-actin.

2. A DNA sequence according to Claim 1, which is chromosomal and includes at least one intron.

3. A DNA sequence of less than about 1000kb including the β-actin transcriptional initiation region.

4. A DNA sequence according to Claim 3 extending downstream not farther than the twelfth nucleotide in the coding region.

5. A DNA sequence according to Claim 4, having downstream from said transcriptional initiation, intron I.

6. A DNA construct comprising a bacterial replication system and a sequence coding for at least one exon of a human β-actin.

7. A construct according to Claim 6, including all of the exons of β-actin.

8. A construct according to Claim 6, wherein said exons are separated by β-actin introns.

9. A DNA sequence coding for at least a substantial proportion of intron I having a flanking region adjacent a terminus of said intron I DNA sequence in the downstream order of transcription coding for other than β-actin.

10. A DNA sequence including introns I, II, III, IV or V of β-actin or fragments thereof retaining the splicing donor and acceptor terminal sequences, each of said introns or fragments substantially free of coding sequences of β-actin.

11. A DNA intron sequence according to Claim 10 flanked by expression sequences, which upon excision of said DNA intron sequence have an open reading frame.

12. A DNA construct comprising a human β-actin transcriptional and translational sequence joined at its 3'-terminus to a DNA sequence coding for a polypeptide other than β-actin either directly or through the intermediary of β-actin intron I, wherein said coding DNA sequence is joined at its 3'-terminus to a transcriptional termination region, with the proviso that said coding sequence may be interrupted by 0 to 4 β-actin introns other than intron I, or fragments thereof capable of excision in a mammalian host.

13. A mammalian cellular host including a DNA construct according to Claim 12.

14. A host according to Claim 13, wherein said host is a primate.

15. A method for obtaining a polypeptide expression product which comprises:

growing a host according to Claim 13; and isolating said polypeptide encoded for by said coding sequence.

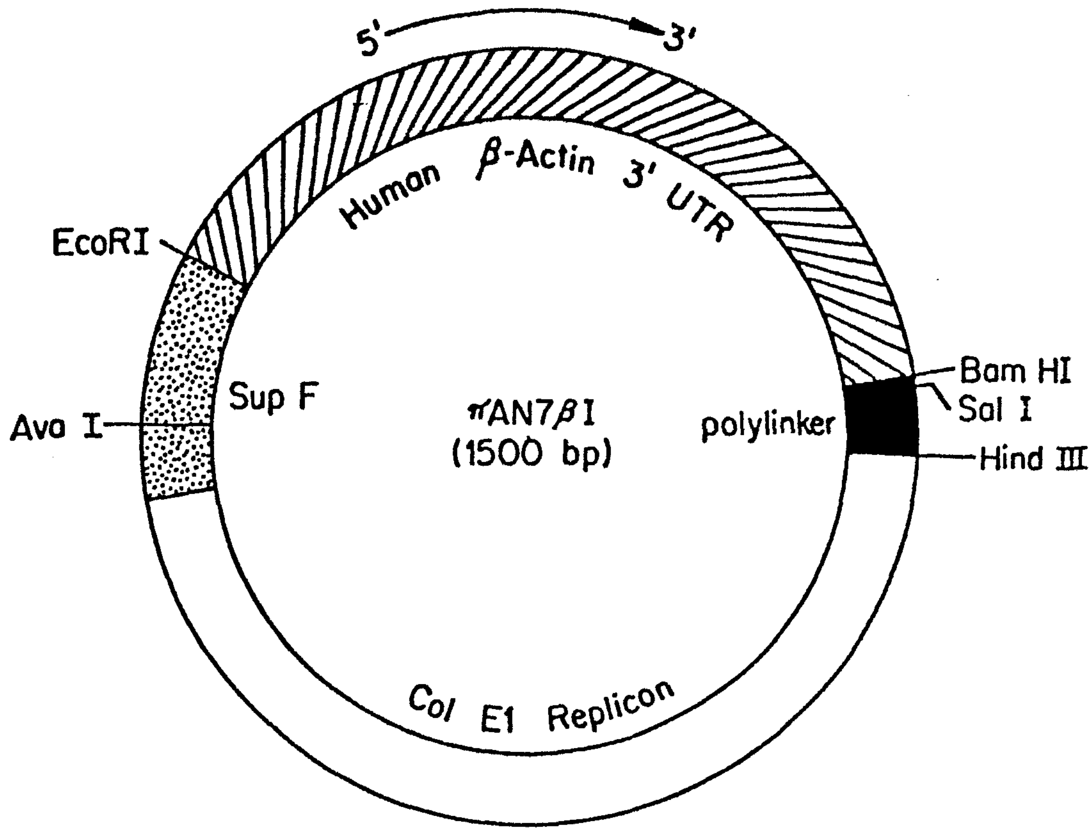
5'
3'
Human β-Actin 3' UTR
EcoRI
Sup F
Ava I
πAN7βI
(1500 bp)
polylinker
Bam HI
Sal I
Hind III
Col E1 Replicon

FIG.—1.

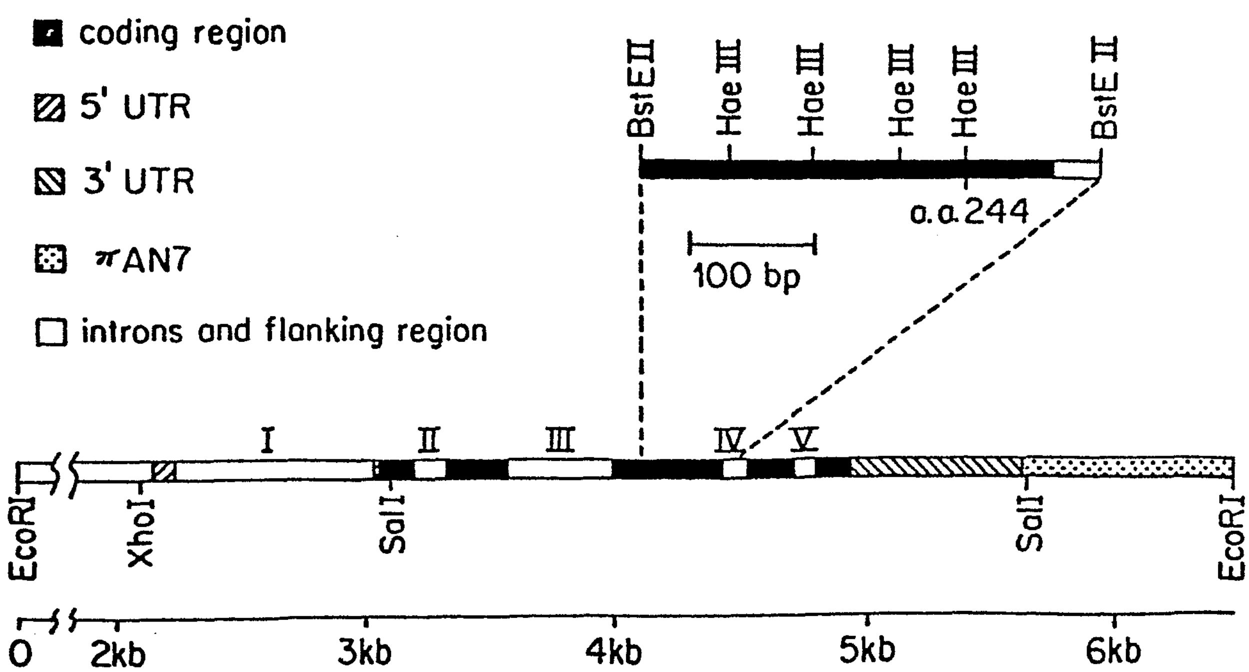
coding region
5' UTR
3' UTR
πAN7
introns and flanking region
BstEII
HaeIII
HaeIII
HaeIII
HaeIII
BstE II
a.a.244
100 bp
I
II
III
IV
V
EcoRI
XhoI
SalI
SalI
EcoRI
0
2kb
3kb
4kb
5kb
6kb

FIG.—2.

European Patent Office

# EUROPEAN SEARCH REPORT

0174608
Application number

EP 85111225.0

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int Cl 4) |
| X,D | NUCLEIC ACIDS RESEARCH, vol. 12, no. 3, February 10, 1984, (Oxford, GB) P. PONTE et al. "Evolutionary conservation in the untranslated regions of actin mRNAs: DNA sequence of human beta-actin cDNA" pages 1687-1696 * Abstract; Fig. 2 * | 1 | C 12 N 15/00<br>C 12 P 19/34<br>C 12 N 5/00<br>C 12 P 21/02<br>C 07 K 13/00<br>C 07 H 21/04 |
| D,A | NUCLEIC ACIDS RESEARCH, vol. 11, no. 6, March 25, 1983, (Oxford, GB) U. NUDEL et al. "The nucleotide sequence of the rat cytoplasmic beta-actin gene" pages 1759-1771 * Abstract; Fig. 2 * | 1,6, 12 | |
| | | | TECHNICAL FIELDS SEARCHED (Int. Cl.4) |
| | | | C 12 N<br>C 12 P<br>C 07 K<br>C 07 H |

The present search report has been drawn up for all claims

| Place of search | Date of completion of the search | Examiner |
|---|---|---|
| VIENNA | 10-12-1985 | WOLF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document